(19) Europäisches Patentamt
European Patent Office
Office européen des brevets

(11) EP 2 895 012 B1

## (12) EUROPEAN PATENT SPECIFICATION

(45) Date of publication and mention of the grant of the patent:
**18.10.2017 Bulletin 2017/42**

(21) Application number: **13763020.8**

(22) Date of filing: **10.09.2013**

(51) Int Cl.:
*A23L 33/105* (2016.01)          *A23L 33/135* (2016.01)
*A61K 35/745* (2015.01)          *A61K 35/747* (2015.01)

(86) International application number:
**PCT/EP2013/068656**

(87) International publication number:
**WO 2014/040962 (20.03.2014 Gazette 2014/12)**

(54) **PREBIOTIC THYLAKOID COMPOSITION**

PRÄBIOTISCHE THYLAKOIDZUSAMMENSETZUNG

COMPOSITION PRÉBIOTIQUE DE THYLAKOÏDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.09.2012 SE 1251019**

(43) Date of publication of application:
**22.07.2015 Bulletin 2015/30**

(73) Proprietor: **Thylabisco AB**
**224 71 Lund (SE)**

(72) Inventors:
• **ALBERTSSON, Per-Åke**
**S-224 71 Lund (SE)**
• **ERLANSON ALBERTSSON, Charlotte**
**S-224 71 Lund (SE)**

(74) Representative: **Ström & Gulliksson AB**
**P.O. Box 4188**
**203 13 Malmö (SE)**

(56) References cited:
**WO-A1-2012/113918          CN-A- 101 278 752**
**US-A1- 2008 175 899**

• **Anonymous: "Greenleaf Medical AB's Appethyl(TM) Named Finalist for Best New Ingredient NutrAward", , 19 February 2013 (2013-02-19), pages 1-2, XP055088965, Retrieved from the Internet: URL:http://www.prweb.com/releases/2013/2/prweb10437786.htm [retrieved on 2013-11-18]**
• **DATABASE BIOSIS [Online] BIOSCIENCES INFORMATION SERVICE, PHILADELPHIA, PA, US; September 2009 (2009-09), LAPOINTE TAMIA K ET AL: "The role of epithelial malfunction in the pathogenesis of enteropathogenic E. coli-induced diarrhea", XP002716562, Database accession no. PREV200900535812 & LABORATORY INVESTIGATION, vol. 89, no. 9, September 2009 (2009-09), pages 964-970, ISSN: 0023-6837, DOI: 10.1038/LABINVEST.2009.69**

SEQUENCE LISTING

<110> Thylabisco AB

<120> PREBIOTIC THYLAKOID COMPOSITION

<130> W87940003

<150> SE1251019-4
<151> 2012-09-11

<160> 10

<170> BiSSAP 1.2

<210> 1

<211> 20
<212> DNA
<213> Lactobacillus

<220>
<221> source
<222> 1..20
<223> /mol_type="unassigned DNA" /organism="Lactobacillus"

<400> 1
ggaatcttcc acaatggacg          20

<210> 2
<211> 23
<212> DNA
<213> Lactobacillus

<220>
<221> source
<222> 1..23
<223> /mol_type="unassigned DNA" /organism="Lactobacillus"

<400> 2
cgctttacgc ccaataaatc cgg          23

<210> 3
<211> 18
<212> DNA
<213> Bifidobacterium

<220>
<221> source
<222> 1..18
<223> /mol_type="unassigned DNA" /organism="Bifidobacterium"

<400> 3
tcgcgtcygg tgtgaaag          18

<210> 4
<211> 17
<212> DNA
<213> Bifidobacterium

<220>
<221> source
<222> 1..17
<223> /mol_type="unassigned DNA" /organism="Bifidobacterium"

<400> 4
ccacatccag crtccac          17

<210> 5
<211> 25
<212> DNA
<213> Enterobacteriaceae

<220>
<221> source

<222> 1..25
<223> /mol_type="unassigned DNA" /organism="Enterobacteriaceae"

<400> 5
cattgacgtt acccgcagaa gaagc        25

<210> 6
<211> 21
<212> DNA
<213> Enterobacteriaceae

<220>
<221> source
<222> 1..21
<223> /mol_type="unassigned DNA" /organism="Enterobacteriaceae"

<400> 6
ctctacgaga ctcaagcttg c       21

<210> 7
<211> 24
<212> DNA
<213> Bacteroides <genus>

<220>
<221> source
<222> 1..24
<223> /mol_type="unassigned DNA" /organism="Bacteroides <genus>"

<400> 7
tttattgggt ttaaagggag ggta        24

<210> 8
<211> 25
<212> DNA
<213> Bacteroides <genus>

<220>
<221> source
<222> 1..25
<223> /mol_type="unassigned DNA" /organism="Bacteroides <genus>"

<400> 8
caatcggagt tcttcgtgat atcta        25

<210> 9
<211> 20
<212> DNA
<213> Escherichia coli

<220>
<221> source
<222> 1..20
<223> /mol_type="unassigned DNA" /organism="Escherichia coli"

<400> 9
agagtttgat nntggctcag        20

<220>
<221> source
<222> 1..20
<223> /mol_type="unassigned DNA" /organism="Escherichia coli"

<400> 10
cggntacctt gttacgactt                20

**Description**

FIELD OF INVENTION

The present invention relates to the field of prebiotics, and especially to a composition stimulating the growth and/or activity of probiotic bacteria, typically lactic acid bacteria, in the digestive system of mammals.

BACKGROUND

Probiotic bacteria are well known in the art, and their health benefits documented. In order to increase benefits of probiotic bacteria, they may be supplemented by prebiotic substances promoting the bacteria. Prebiotics are non-bacterial products with a beneficial effect on the microbiota. They act as fertilizers to the colonic microbiota, thereby enhancing growth of beneficial commensal organisms, such as *Bifidobacterium* and *Lactobacillus.* A prebiotic is typically a selectively fermented ingredient that allows specific changes, both in the composition and/or activity in the gastrointestinal microflora, also known as the gut microbiota or commensal flora, that confers benefits upon host well-being and health.

Promotion of probiotic bacteria, such as typically lactic acid bacteria, e.g. *Lactobacilli* and *Bifidobacterium,* may be beneficial in the treatment of diseases associated with disturbed gut microbiota. As example, antibiotic-associated diarrhea, travellers' diarrhea, pediatric diarrhea, and irritable bowel syndrome are conditions known to be associated with disturbed gut microbiota. Also, use of prebiotic agents has been associated with a reduced risk for colorectal cancer. Furthermore, disturbances in the commensal flora can occur due to a variety of other factors including, but not limited to, radiation therapy or accidental exposure, use of antibiotics and other agents influencing bacterial growth, diseases of the gut such as intolerance to gluten. Such unwanted changes can give rise to disease and disturbed immune function and be rectified by the use of prebiotics so that normal physiological growth and function of both the commensal flora and the host can be achieved.

Thus, conditions where a beneficial gut bacterial flora is disturbed may be treated or prevented by promoting probiotic bacteria.

A vast variety of potential prebiotic substances exist. As an example, in EP 1 056 358 B1 it is described that oral intake of a specific monosaccharide in a prebiotic food stimulates the growth of beneficial bacteria in the human colon. US 2008/175899 A1 describes an orally administered composition for promoting nasal sinus health comprising at least one probiotic agent in combination with N-acetylcysteine (NAC) and a bioflavonoid or derivate thereof.

WO 2012/113918 A1 describes a composition comprising thylakoids, or parts thereof, for reducing the transport of molecules through the intestinal mucosa of the intestinal tract of a mammal.

CN 101 278 752 A describes a series of instant carnitine natural fruit-vegetable beverage dry powder prescriptions and a manufacturing technique thereof.

However, there is a need for finding new and improved prebiotic substances with added benefits, which are easy to produce and safe to ingest.

SUMMARY

Accordingly, the present invention preferably seeks to overcome the above-identified deficiencies in the art singly or in any combination and solves at least the above mentioned problems by providing a new and improved prebiotic composition with added benefits, which is easy to produce and safe to ingest.

According to an aspect of the invention, non-therapeutic use of a composition comprising isolated, enriched thylakoids, or parts thereof, as prebiotic agent is provided. Such use is typically for promoting lactic acid bacteria, such as *Lactobacilli* and *Bifidobacterium,* in the intestines, such as ileum and caecum, e.g. ileum, of a mammal.

According to another aspect of the invention a composition comprising isolated, enriched thylakoids, or parts thereof, and probiotic bacteria, wherein the chlorophyll content in the composition is from 1 mg to 75 mg chlorophyll per g composition is provided.

Further advantageous features of the invention are defined in the dependent claims. In addition, advantageous features of the invention are elaborated in embodiments disclosed herein.

BRIEF DESCRIPTION OF DRAWINGS

These and other aspects, features and advantages of which the invention is capable of will be apparent and elucidated from the following description of embodiments of the present invention, reference being made to the accompanying drawings, in which

Fig. 1 is a graph showing concentration (pM) of plasma insulin according to an embodiment;

Figs 2 and 3 are graphs showing viable count (CFU/g tissue) of Lactobacilli according to embodiments of the invention;

Fig. 4 is a graph showing viable count (CFU/g tissue) of bacteria according to an embodiment; and

Figs 5 and 6 are graphs showing principal component analysis (PCA) of T-RFLP data according to embodiments of the invention.

DETAILED DESCRIPTION

Several embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

Thylakoids may be extracted from the chloroplast membrane of green leaves and contain proteins, lipids and pigments (e.g. chlorophyll and carotenoids). They have been found to increase satiety, decrease hunger signals and promote weight loss in several studies, both in human and animal subjects (cf. WO 2006/132586 and WO 2010/008333 among others). The satiety-promoting effects are explained in two ways: Thylakoids interact with dietary triacylglycerides and the lipase/colipase complex and, as a result, cause a physical hindrance, thereby prolonging the digestion of the dietary fat and also the uptake of nutrients over the intestinal wall.

Thylakoids are digested by the body, why they can be used without any toxic effects. However, thylakoids are digested more slowly than other cell compartments, such as mitochondria, which is beneficial. As recognized by the skilled person, a prebiotic agent is an ingredient that allows specific changes, both in the composition and/or activity in the gastrointestinal microflora that confers benefits upon host well-being and health.

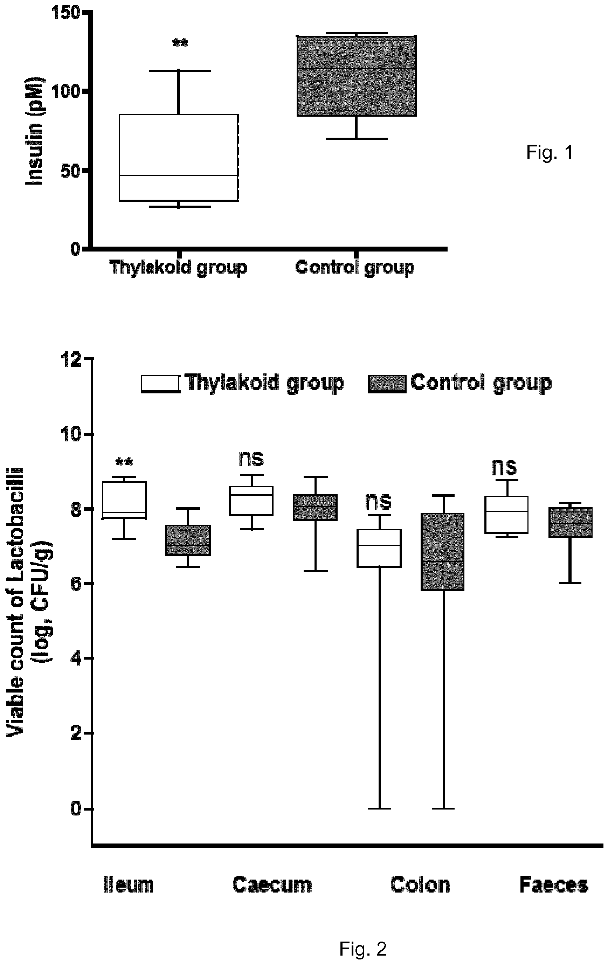

The inventors investigated the effects of thylakoids on the gut microbiota and the outcome after a glucose-tolerance test in rats. They surprisingly found that thylakoids regulate gut microbiota (cf. FIG 2 to 4), hence function as a prebiotic agent. Further, they found that thylakoids increase the insulin sensitivity (cf. FIG 1).

In short, Sprague-Dawley rats were divided in two groups and fed either a thylakoid enriched diet or a control diet, of normal rat chow, for 10 days. After 10 days a glucose-tolerance test was performed, resulting in significant lower concentration of insulin in the thylakoid-fed rats compared to control (cf. FIG 1). Analysis of bacteria showed a significant increase of Lactobacilli in ileum, induced by the addition of thylakoids, demonstrated both with bacterial culture of intestinal samples and by qPCR. Also a significantly reduced amount of faecal lactobacilli was observed by qPCR analysis in thylakoid group, suggesting that lactobacilli might be more adherent to the mucosa of the intestine after supplementation of thylakoids for 10 days.

Analysis of other bacteria indicated that Gram-positive bacteria were significantly decreased in colon, caecum and faeces for the thylakoid group compared to control. Analysis of faecal microbiota through principal component analysis of terminal restriction fragment length polymorphism (T-RFLP) demonstrated distinct microbial populations when comparing thylakoids and control animals. Taken together, these findings indicate that thylakoids may regulate gut microbiota, hence function as a prebiotic agent. This in turn might be important in regulation of body weight and metabolic disorders.

The present inventors have thus found that oral administration of a composition comprising thylakoids, or parts thereof, to a mammal enhances the intestinal probiotic flora and promotes lactic acid bacteria. A composition comprising isolated, enriched thylakoids may thus function as a prebiotic agent. Furthermore, various probiotic strains, especially Lactobacilli, have been shown to be useful in preventing or treating impaired immune function, , and antibiotic-associated diarrhea, travellers' diarrhea, pediatric diarrhea, and irritable bowel syndrome.

According to an embodiment, a composition comprising isolated, enriched thylakoids, or parts thereof, is thus provided to promote lactic acid bacteria, such as *Lactobacilli* and *Bifidobacterium,* in the intestines of a mammal. The mammal may be a human being. Typically, the composition is administrated orally in order to reach the intestines. By promoting lactic acid bacteria in the intestines, and especially the ileum and caecum, more preferably the ileum, conditions associated with disturbed gut microbiota, including, but not limited to, impaired immune function,, and antibiotic-associated diarrhea, travelers' diarrhea, pediatric diarrhea, and irritable bowel syndrome, may be prevented or treated.

Although, isolated, enriched thylakoids, or parts thereof may be used to regulate the gut microbiota, probiotic bacteria is, according to an embodiment, included in the composition. By including probiotic bacteria, the effect on the gut microbiota may be enhanced and the existing probiotic bacteria complemented. The probiotic bacteria to be included in the composition may be *Bifidobacterium,* e.g. *Bifidobacterium infantis,* and *Lactobacillus,* e.g. *Lactobacillus plantarum.*

The general principles of isolating and enriching thylakoids are known in the art (cf. PCT/SE2006/000676, WO/SE2009/000327 and Emek, SC, Szilagyi A, Akerlund H E, et al. (2010) A large scale method for preparation of plant thylakoids for use in body weight regulation Prepar biochem & biotech 40 (1), 13-27.).

Typically, the thylakoids of the present composition are enriched from green leaves or green algae. Preferably, the thylakoids are isolated and enriched from spinach. Before adding the thylakoids to the composition, they may be concentrated in a manner such that the chlorophyll content of the isolated, enriched thylakoids is from about 8 mg to 150 mg chlorophyll per g. The content of thylakoids in the composition may correspond to a chlorophyll content of 1 mg to 75 mg chlorophyll per g composition.

The composition used to promote lactic acid bacteria, such as *Lactobacilli* and *Bifidobacterium,* in the intestines, may be a foodstuff or a medicament. According to an embodiment, the composition is an oral pharmaceutical or nutraceutical composition comprising a physiologically tolerable oil-in-water emulsion comprising thylakoids. In such an embodiment, the amount of thylakoids is 20 to 30 wt.%, such as about 25 wt.%. Further, the oil-content may be 20 to 30 wt.%, such as about 25 wt.%. The oil used may be vegetable oils of food grade, such as rape seed oil, olive oil, sunflower oil etc.

As the composition may be administered in various forms, the daily dose of thylakoids to be administered may vary. As guiding principle, the amount of the composition to be administered may be selected in manner such that the dosage correlates to a daily dose of 2.4 mg to 240 mg thylakoids per kg body mass of the mammal.

According to another embodiment, the composition of thylakoids disclosed herein is provided for non-therapeutic use as a prebiotic agent. As the composition of thylakoids disclosed herein may change both the composition and/or the activity of the gastrointestinal microflora it may confer benefits upon host well-being and health.

The probiotic bacteria normally residing in the human gastrointestinal tract, collectively referred to as the gut microbiota, has an important role in nutrient acquisition and energy regulation. In recent years, it has been proposed that the microbiota is different between normal weight persons and overweight or obese persons. Numerous findings from human and animal studies have revealed that the quantity of two dominant divisions of gut bacteria, namely the gram positive *Firmicutes* and the gram negative *Bacteroidetes,* are closely related to the pathophysiology of obesity by altering the host's energy-harvesting efficiency. Accordingly, in obese animals and patients, an increase of *Firmicutes* and reduction of *Bacteroidetes* in the gut has been demonstrated. An opposite shift in the ratio of these specific bacteria occurs as obese patients lose weight on low-calorie diets. On the other hand, evidence about such variations of intestinal microbiota in obese humans was contradictory. Regarding the *Firmicutes* and the *Bacteroidetes,* there were found no differences between obese and non-obese individuals and even though in obese in comparison with normal weight peoples a significant abundance of *Bacteroidetes* has been documented. These findings raise the possibility that the gut microbiota plays an important role in regulating body weight and may be a factor in the development of obesity.

Other metabolic diseases, as diabetes, are now also being demonstrated to be associated with compositional changes of the microbiota. It has also been proposed that a dys-regulated intestinal barrier, a leaky gut, might lead to insulin resistance and eventually the development of diabetes. To reinforce the intestinal barrier would accordingly be a new therapeutic way to prevent, and maybe also treat, diabetes and prevent obesity.

Ingestion of low-glyceamic foods, such as fibres, that referred to as prebiotics is a well-accepted as a way to enhance satiety by slowing down the intestinal digestion of carbohydrates. Prebiotics is a non-bacterial product with a beneficial effect on the microbiota. They act as fertilizers to the colonic microbiota, thereby enhancing growth of beneficial commensal organisms such as *Bifidobacterium* and *Lactobacillus.* However, the satiety signals from the prolonged digestion of carbohydrates are obviously not enough.

Today, over one billion adults are overweight, that is having a body mass index (BMI) between 25-30 kg/m$^2$, and more than 300 million are obese, with BMI > 30 kg/m$^2$, worldwide. Moreover, overweight and obesity are strongly associated with hyperlipidemia, atherosclerosis and cardiovascular disease.

Nowadays we have an increased availability of palatable food (i.e. food rich in fat and sucrose) in the western society. This may contribute to the observed global increase in body weight over the past decades. To help individuals suffering from overweight and obesity, it is therefore crucial to find a way to strengthen satiety signals and dampen hunger signals. We have found that after dietary supplementation of thylakoid membranes from green leaves to food, several appetite hormones, such as ghrelin and cholecystokinin (CCK) (unpublished data) as well as insulin and leptin are affected.

Without further elaboration, it is believed that one skilled in the art may, using the preceding description, utilise the present invention to its fullest extent. The following preferred specific embodiments are, therefore, to be construed as merely illustrative and not limitative of the remainder of the disclosure in any way whatsoever.

Although the present invention has been described above with reference to (a) specific embodiment(s), it is not intended to be limited to the specific form set forth herein. Rather, the invention is limited only by the accompanying claims and, other embodiments than the specific above are equally possible within the scope of these appended claims, e.g. different than those described above.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous.

In addition, singular references do not exclude a plurality. The terms "a", "an", "first", "second" etc do not preclude a plurality.

EXPERIMENTAL

The aim of the experimental work described below was to study the effects of thylakoids on gut microbiota in healthy rat, and also their effects on blood insulin. The composition of the microbiota was analysed by both cultural and culture-independent techniques. Viable count of specific bacteria based on their metabolic requirements as the cultural technique, and quantitative PCR (q-PCR), terminal restriction fragment length polymorphism (T-RFLP) and 16S rDNA sequencing as the culture-independent techniques.

However, the following examples are mere examples and should by no mean be interpreted to limit the scope of the invention. Rather, the invention is limited only by the accompanying claims.

## Animals & experimental procedures

The study was performed on rats (*Rattus norvegicus*) of the Sprague-Dawley strain (Mol: SPRD Han; Taconic M & B A/S, Ry, Denmark), bred under specific pathogen-free condition with a controlled environment (20°C $\pm$ 1°C, 50% $\pm$ 10% relative humidity, 12:12-hour light dark cycle) and using an open cage system. After 7 days of acclimatization, totally 16 rats were kept individually and fed either a control diet (8 rats) or a thylakoid-enriched diet (8 rats) for 10 days.

Thylakoid membranes were extracted and purified from fresh baby-spinach leaves as described before (Emek, SC, Szilagyi A, Akerlund H E, et al. (2010) A large scale method for preparation of plant thylakoids for use in body weight regulation Prepar biochem & biotech 40 (1), 13-27; Montelius C, Gustafsson K, Westrom B, et al. (2011) Thylakoids reduce glucose uptake and decrease intestinal macromolecular permeability. Br J Nutr 106 (6), 836-44).

Standard rat chow (R 36, Lantmännen, Stockholm, Sweden) was enriched with either only rapeseed oil (control diet), or a thylakoid-oil suspension (thylakoid diet). The thylakoid diet was prepared by mixing 4 g thylakoid powder (corresponding to 132 mg chlorophyll), 5 g of rapeseed oil and 10 g water by using an Ultraturrax mixer. The amount of thylakoids supplemented was calculated by the addition of 6 mg chlorophyll per gram of normal food intake (22 g food / rat / day). Both diets were isocaloric, and had the energy (E) distribution of 25 E% carbohydrates, 60 E% fat and 15 E% proteins.

Bodyweight was recorded every day during the 10-day experimental time. In the morning, 25 g of the normal rat chow was given to every rat for free eating during the day. In the following afternoon the remaining rat chow was removed and the amount eaten was measured. Each rat was then given 15 g of the experimental diet. On the 11th day faecal samples were collected under specific pathogen-free conditions in the morning, before a glucose tolerance test was performed.

A 15% glucose solution with a volume of 10% of their body weight was given as a bolus-dose. The concentration of plasma glucose was measured by a prick in the tail at time points 0, 15, 30, 45, 60, 90 and 120 min, whereas insulin was measured only after 120 minutes. The rat was then anesthetised with isoflouran (Schering- Plogh a/s, Ballerup, Denmark) before a laparotomy was performed. Intestinal mucosal samples from the ileum, caecum and colon were collected and immediately placed in sterile tubes containing 3 ml freezing media. The study was approved by the Lund University Ethical Review Committee for Animal Experiments and conducted according to the European Community regulations concerning the protection of experimental animals.

## Analysis of bacterial counts

Conventional dilution procedures were used for the viable count of lactobacilli, bifidobacteria and Enterobactericeae in the intestinal mucosal samples from ileum, caecum and colon, as well as from the faecal samples. Samples from appropriate dilutions were plated on Rogosa agar, modified Wilkins-Chalgren (MW) agar and violet red bile glucose agar (all from Oxoid, Malmö, Sweden), and incubated at 37 °C for 72 h anaerobically and 24 h aerobically before colony forming units (CFU) per gram tissue or feaces were calculated.

## 16S rDNA sequencing

Totally 169 isolates (4-6 from every rat) from colon and ileum samples were randomly picked for 16S rDNA sequencing (MW agar plates:52 colon isolates, rogosa plates: 53 colon isolates, Rogosa plates: 64 ileum isolates). As template for the polymerase chain reaction (PCR), crude cell extracts were used. 16S rRNA genes were amplified by using the forward primer ENV1 and the reverse primer ENV2 (Table 1), (Applied Biosystems, Stockholm, Sweden). The PCR reaction mixture of totally 25 $\mu$l contained 0·4 $\mu$M primer ENV1, 0·2 $\mu$M primer ENV2, 2·5 $\mu$l 10 x PCR reaction buffer (500 mM Tris-HCl, 100 mM KCl, 50 mM $(NH_4)_2SO_4$, 20 mM $MgCl_2$, pH 8·3), 0·2 $\mu$M deoxyribonucleotide triphosphate, 2·5 U of FastStart Taq DNA polymerase (Roche Diagnostics, Mannheim, Germany), and 2 $\mu$l of template DNA. The PCR was performed in an Eppendorf Master Cycler (Eppendorf, Hamburg, Germany) using the following program: 95 °C for 3 min, 94 °C for 3 min, 30 cycles of 94 °C á 1 min, 50 °C for 45 sec and 72 °C for 2 min, followed by an additional extension of 72 °C for 7 min. PCRs containing only reagents without sample DNA were run in parallel as negative PCR controls. PCR products (2 $\mu$l) were verified on 1·5% agarose gel. The amplification products were sent in 96-well plates for sequencing at MWG-Biotech (Ebersbery, Germany). The generated sequences were compared to the GenBank database (National Center for Biotechnology Information, Rockville Pike, Bethesda, MD, USA)

DNA extraction of faeces and intestinal biopsies

Faecal and intestinal segments of ileum and colon were thawed and placed in an ultrasonic bath (Millipore, Sundbyberg, Sweden) for 5 min and vortex for 2 min before centrifuged (Eppendorf 5804R, Hamburg, Germany) at 12851 g for 10 min. The supernatant was discarded and 190 $\mu$l buffer G2 and 15 $\mu$l Proteinase K (DNA Tissue Kit; Qiagen, Hilden, Germany) were added to the intestinal samples. PBS buffer (500 $\mu$l/ 50 mg) was added to the faecal samples before all samples were incubated at 56°C over-night in a shaking water bath. After centrifugation (Eppendorf 5804R) at 12851 g for 8 min, the solution was transferred to a Qiagen sample tube. Total DNA was extracted and eluted in 200 $\mu$l buffer using Biorobot EZ1 (Qiagen) according to the manufacturer's instructions.

Quantitative PCR (qPCR) Analysis

Bacterial groups were estimated using separate quantitative PCR assays. Each assay reaction contained 10$\mu$l QuantiTect® SYBR Green PCR Master Mix (Qiagen), 0·5 $\mu$M of each primer (table 1), 2 $\mu$l of template DNA and RNase-free water to reach a final volume of 20 $\mu$l. Samples, standards and non-template controls were run as duplicates. The thermal cycling was carried out in Rotor-Gene Q (Qiagen) with a program of 95°C for 15 min, followed by 40 cycles with denaturation at 95°C for 15 sec, annealing at 56°C to 60°C for 30 sec and elongation at 72°C for 30 sec. The fluorescent products were detected at the last step of each cycle. Melting curve analysis was made to ensure specific amplification. Absolute abundance of 16S rRNA genes was calculated based on standard curves using Rotor-Gene Q series (Software 1.7, Qiagen). Detection limit was $10^2$ copies/ reaction for all assays. To construct standard curves, cloned products from *Lactobacillus plantarum* DSM9843, *Bifidobacterium infantis* DSM15159, *Escherichia coli* CCUG29300 and *Bacteroides prevotella* were used. One loop of the cell suspension (cloned products) was transferred to 10 ml of lysogenic broth (LB) medium with ampicillin and incubated overnight at 37°C. DNA extraction was performed by using QIAprep® (Miniprep kit, Qiagen). The concentration of DNA (ng/$\mu$l) was finally measured with Nanodrop ND-1000 (Saveen Werner AB, Malmö, Sweden), and used for copy number calculation. Tenfold dilution series of the DNA products were made in TE buffer (10 mM Tris, 1mM EDTA, pH 8·0). Numbers of bacteria were expressed as numbers of amplicon copies/g wet weight of faeces or tissue.

Terminal restriction fragment length polymorphism (T-RFLP) analysis

16S rRNA genes were amplified as described above with the exception that the forward primer ENV1 was fluorescently labelled with a fluorescent dye (FAM) at the 5' end. Triplicate reactions were carried out for each sample and a negative control was included in all PCR runs. PCR products were verified on a 1·5 % agarose gel. PCR products of each sample were pooled and further purified by using the MinElute PCR purification Kit (Qiagen). DNA was finally eluted in 15 $\mu$l of PE buffer and DNA concentration measured by Nanodrop ND-1000 (Saveen Werner AB).

T-RFLP Analysis

Aliquots (200 ng) of purified PCR products were digested at 37°C (Mastercycler® 5333, Eppendorf) with either the restriction endonuclease enzyme *Msp*I for 5 hours or the restriction endonuclease enzyme *Alu*I for 2 hours (Fermentas, St. Leon-Rot, Germany). Inactivation was made by heating at 65°C for 20 minutes. After digestion, aliquots of the products were diluted 4 times with sterile water in a 96-well plate. Samples were then sent to the DNA-lab (Skåne University Hospital, Malmö, Sweden) for T-RFLP analysis in a capillary electrophoresis system. The data from the electrophoresis were analysed with GeneMapper® (version 4.0, Applied Biosystems, Foster city, CA, USA) and the fragments sizes and peak areas were estimated using the Southern method (GeneMapper®). The size range was set from 30 base pairs (bp) to 600 bp. The peak amplitude threshold was set to 50 relative fluorescence units (rfu) for samples and 10 rfu for the internal standard. The total peak area for each sample was calculated by summarizing the area for all peaks in a sample. The individual relative peak area was expressed as percentage of the total area.

Calculations

Shannon-Wiener index (H) was calculated using relative peak area expressed as percentage of the total area for a sample by using the equation:

$$H'=-\sum pi\ lnpi$$

Where *pi* is the relative area expressed as percentage and *In* is the **natural logarithm.** Multivariate data analysis with principal component analysis (PCA) was performed in SIMCA-P+ (12.0.1, Umetrics, Umeå, Sweden) to reveal the possible differences in microbial population between the groups.

Statistics

Values are presented as median $\pm$ SEM or as 10th, 25th, 75th, and 90th percentiles. The differences between the two experimental groups were assessed by a Mann-Whitney rank sum test and/or *t-test* using Sigma-Stat 3.0 (SPSS Inc., Chicago, Il, USA) and GraphPad 4.0, (Graphpad Inc, La Jolla, CA, USA). The incidence of the bacteria was evaluated by the Fisher exact test (Quick-Stat version 2.6, Sapio HB, Sweden). $P < 0.05$ were considered significant.

Results

Bodyweight, plasma glucose and insulin

All rats finished their experimental diets during every night of the study. No significant differences were found between the thylakoid and control groups concerning bodyweight or intake of normal rat show during the 10-days experiment (data not shown). The plasma-insulin concentration at 120 minutes after the bolus-dose was however significantly lower for the thylakoid group compared to the control, as seen in Fig. 1. Here, the concentration (pM) of plasma insulin 120 minutes after a bolus dose of glucose was given to the rats is shown. The insulin levels were significantly lower in the thylakoid group compared to the control group (p= 0·0082). However, no difference between the groups was found for the plasma glucose concentrations.

Bacterial quantification & identification

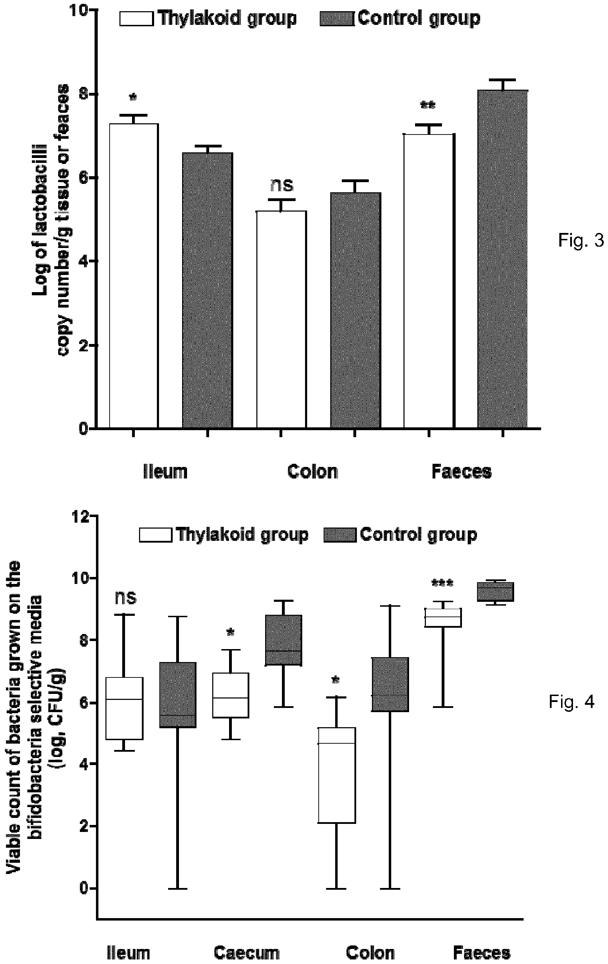

Both the viable count, as seen in Fig. 2, and the qPCR analysis, as seen in Fig. 3, for lactobacilli were significantly increased in the ileal mucosa in the thylakoid group compared to control. Fig. 2 shows the viable count (CFU/g tissue) of Lactobacilli in the mucosa of ileum, caecum, colon and in faeces. The amount of Lactobacilli in the thylakoid group was significantly increased in the ileum compared to the control group (p= 0·007), whereas no difference was seen regarding caecum, colon and faeces; and Fig. 3 shows the qPCR analyses of the content of lactobacilli in the mucosa of ileum, colon and in faeces. The lactobacilli was significantly increased in ileal mucosa (p=0·032) and significantly decreased in the faeces (p=0·007) for the thylakoid group compared to the control group. No difference was observed in the colonic mucosa. No difference was observed in the mucosal samples of colon or ceacum for both methods. However, the qPCR, but not the viable count, showed a significant decrease of lactobacilli in the fecal samples for the thylakoid group, as seen in Fig. 3. The melting curve analysis of the qPCR demonstrated that faecal and mucosal lactobacilli were different (data not shown). 16S rDNA sequencing for isolates picked randomly from Rogosa plates showed a significantly higher incidence of *Lactobacillus reuteri* in ileum for the thylakoid group compared to control. A tendency of increased incidence of *Lactobacillus reuteri* with thylakoids was also observed in colon, as well as a significantly lower incidence of *Lactobacillus johnsonii* in ileum and colon (Table 2).

Analyses of Bifidobacteria by qPCR showed a similar content for both groups in the mucosa of the ileum, colon and also in the faeces (data not shown). For the viable count a significant decrease of bacteria grown on the bifidobacteria-selective media was found for the thylakoid group. However, identification with 16S rDNA showed that other Gram positive bacteria, and not Bifidobacteria, was more pronounced on the agar plates (bifidobacteria represent less than 25%). Hence, gram positive bacteria as a group was significantly decreased in the thylakoid group compared to control in both the mucosa of ceacum, colon and in feaces, as seen in Fig. 4. Here, the viable count (CFU/g tissue) of bacteria grown on the bifidobacteria selective media in the mucosa of ileum, caecum and colon, and in faeces is shown. The amount of bacteria was significantly decreased in the thylakoid group in caecum (p= 0·0104), colon (p=0·0207) and faeces (P= 0·0006), compared to control group. These Gram positive bacteria were identified as *Staphylococcus spp, Kocuria* and *Bacillus Simplex* (data not shown).

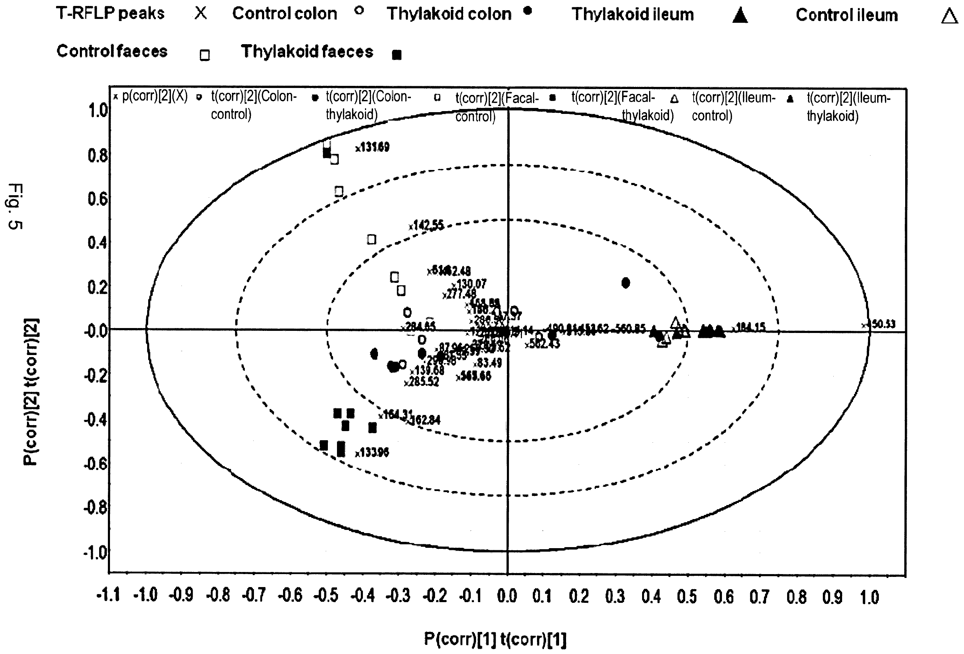

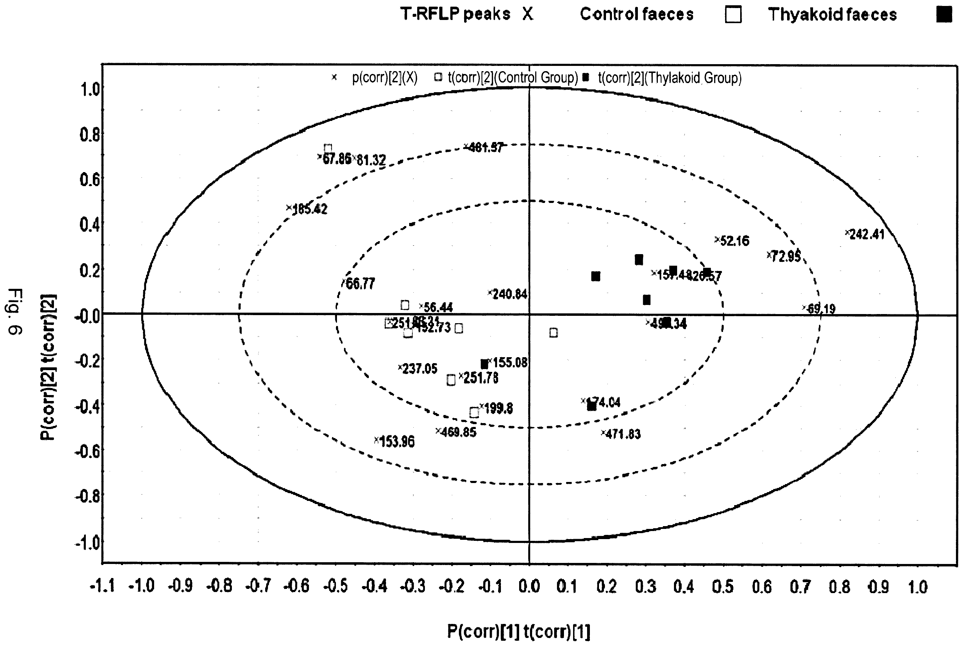

The T-RFLP analysis of mucosal samples from ileum and colon, with the restriction endonuclease *MspI,* resulted in no difference in bacterial groups between the groups, as seen with principal component analysis (PCA). In the faecal samples however, specific bacterial groups were completely separated from each other for the thylakoid group compared to the control, as seen in Fig. 5. Here, principal component analysis (PCA) of T-RFLP data from *Msp*I digestion of colon, and ileum mucosa and faeces in control and thylakoid groups is shown. In the faecal samples (□ = faeces from the control group, ■ = faeces from the thylakoid group) a difference can be seen between specific bacterial groups, which are completely separated from each other. Also by the restriction endonuclease *AluI,* shown by PCA as well, separated bacterial communities were found in the faecal samples from the thylakoid and control groups respectively, as seen in Fig. 6. Here, a principal component analysis (PCA) of T-RFLP data from *Alu*I digestion of faecal samples in control and thylakoid groups is seen. A difference between faecal samples from the control (□) and thylakoid (■) groups can be seen, as they are separated from each other.

No significant differences between the thylakoid and control groups were observed in the analyses of *Enterobacteriaceae* and *Bacteroides* by both viable count and qPCR (data not shown), nor for the bacterial diversity after calculations by the Shannon-Wiener diversity index (data not shown).

Discussion

This study resulted in two main findings. First, supplementations of thylakoids to the diet for ten days affect the insulin sensitivity in rats. A lower concentration of insulin was found for the thylakoid-fed rats in the blood 120 minutes after a bolus-dose of glucose was given, even though no differences of plasma-glucose concentrations was observed between the diets. In using diffusion chambers, an *in vitro* model for uptake and permeability of the intestinal wall, thylakoids have been shown to prolong the uptake of methyl-glucose in a dose-dependent way, as well as causing a sterical hindrance of thylakoids covering the mucosal side of the intestine. A prolonging of the glucose uptake *in vivo* would hypothetically result in a lower concentration of insulin secreted. However, no difference in blood-glucose was observed in this study but still the insulin concentration after 120 min was significantly lower for the thylakoid group. In recent years it has been proposed that a "leaky gut", i.e. an increased permeability of the intestine, can lead to insulin resistance and eventually to the development of diabetes. One explanation to the lowered concentrations of insulin might therefore be the sterical hindrance caused by thylakoids, causing the intestinal wall to be reinforced. This phenomena needs to be further investigated, as it could open up to new therapeutic ways to prevent, and maybe also treat, diabetes mellitus.

Secondly, thylakoids modulate the microbiota in several ways. The concentrations of Lactobacilli in ileum increased, and in the same time Lactobacilli were decreased in the feaces, suggesting that thylakoids cause an increased ileal colonisation of Lactobacilli. It is known that strains of lactobacilli have the ability to colonise the mucosa in high concentrations, supporting our finding. A common subspecies of lactobacilli in the rat is *Lactobacillus reuteri,* which was specifically increased in the thylakoid group compared to control. Moreover, deviated colonization of Gram-positive bacteria was observed in colon and faeces for the thylakoid group, however concentrations of bifidobacteria did not differ between the groups. Thylakoids modulation of the microbiota was also seen by the T-RFLP analysis as distinct groups of bacterial populations was found in the thylakoid group but not in the control.

Previous studies have reported that some strains of *Lactobacillus* and *Bifidobacterium* may be important for body weight regulation, by acting as an anti-obesity factor. Also, overweight subjects have been proposed to have an increased growth of some Gram positive bacteria, as *Stapholycoccus aureus,* compared to lean subjects. Elimination of harmful Gram positive bacteria, and the overall composition of the bacterial flora, appears thus to be important for energy balance. Moreover, it has been shown that *Lactobacillus reuteri* has health-promoting effects restricting growth of harmful bacteria, and other lactobacilli species, as *Lactobacillus rhamnosus* GG and *Lactobacillus gasseri,* have been found to reduce adiposity and inflammation associated with obesity. The observed decrease of potentially harmful bacteria as *Staphylococcus species, Kocuria* and *Bacillus simplex* can either be a direct effect of thylakoids, or as an second effect of the increased colonization of *Lactobacillus reuteri.*

All the above findings show that the addition of thylakoids to the diet for ten days in rats effect the microbiota in several ways. Since the composition of the microbiota has been suggested to be involved in the management of body weight, and thylakoids earlier have been shown to reduce body weight, it can be hypothesised that some effects of the weight loss seen in earlier studies can be due to deviations of the microbiota.

To conclude, we propose that the present findings indicate that thylakoids regulate the gut microbiota, leading to an increased colonisation of Lactobacilli, and a decreased growth of potentially harmful Gram positive bacteria. Thus, thylakoids are a prebiotic agent. Thylakoids also increase the insulin sensitivity, possibly by reinforcing the intestinal barrier function.

**Table 1** - primers used in an embodiment.

| Target group | Sequence (5'-3') | Amplicon size (bp) |
|---|---|---|
| *Lactobacillus* | Lact-16S-F: GGAATCTTCCACAATGGACG **(SEQ ID 1)** | 216 |
| | Lact-16S-R: CGCTTTACGCCCAATAAATCCGG **(SEQ ID 2)** | |
| *Bifidobacterium* | Bifido-F: TCGCGTCYGGTGTGAAAG **(SEQ ID 2)** | 243 |
| | Bifido-R: CCACATCCAGCRTCCAC **(SEQ ID 4)** | |
| *Enterobacteriaceae* | Eco1457-F: CATTGACGTTACCCGCAGAAGAAGC **(SEQ ID 5)** | 195 |
| | Eco1652-R: CTCTACGAGACTCAAGCTTGC **(SEQ ID 6)** | |
| *Bacteroides prevotella* | qBacPre-F: TTTATTGGGTTTAAAGGGAGGGTA **(SEQ ID 7)** | 166 |
| | qBacPre-R: CAATCGGAGTTCTTCGTGATATCTA **(SEQ ID 8)** | |
| *16S rRNA genes*<br>(ENV1, ENV2) | E-coli 8-27-F: AGAGTTTGATIITGGCTCAG **(SEQ ID 9)** | |
| | E-coli 1511-1492-R: CGGITACCTTGTTACGACTT **(SEQ ID 10)** | |

**Table 2.** Incidence of different *Lactobacillus* spp. found by 16S rDNA sequencing of isolates picked randomly from Rogosa plates of ileal and colonic mucosal samples. P < 0.05 was considered a significant increase in the thylakoid group compared to control.

| | Control Group | | Thylakoid Group | | |
|---|---|---|---|---|---|
| | Counts | % | Counts | % | p-value |
| **ILEUM** | | | | | |
| *Lactobacillus reuteri* | 11/29 | 37.9 | 28/31 | 90 | 0.03 |
| *Lactobacillus johnsonii* | 17/29 | 58.6 | 3/31 | 10 | 0.003 |
| *Lactobacillus gasseri* | 1/29 | 3.4 | 0/31 | 0 | ns |
| *Lactobacillus ruminis* | 0/29 | 0 | 0/31 | 0 | ns |
| **COLON** | | | | | |
| *Lactobacillus reuteri* | 10/25 | 40 | 21/25 | 84 | 0.09 |
| *Lactobacillus johnsonii* | 13/25 | 52 | 2/25 | 8 | 0.01 |
| *Lactobacillus gasseri* | 0/25 | 0 | 1/25 | 4 | ns |
| *Lactobacillus ruminis* | 2/25 | 8 | 1/25 | 4 | ns |
| ns=not significant | | | | | |

**Claims**

1. Non-therapeutic use of a composition comprising isolated, enriched thylakoids, or parts thereof, as prebiotic agent.

2. The use according to claim 1, wherein said use is for promoting lactic acid bacteria in the intestines, such as the ileum, of a mammal.

3. The use according to claim 2, wherein said bacteria to be promoted are *Lactobacilli* and/or *Bifidobacterium.*

4. The use according to any one of the claims 1 to 3, wherein the composition further comprises probiotic bacteria, such as *Bifidobacterium,* e.g. *Bifidobacterium infantis,* or *Lactobacillus,* e.g. *Lactobacillus plantarum.*

5. The use according to any of the preceding claims, wherein the thylakoids, or parts thereof, are enriched from green leaves or green algae.

6. The use according to claim 5, wherein said thylakoids, or parts thereof, are thylakoids, or parts thereof, enriched from spinach.

7. The use according to any one of the preceding claims, wherein the chlorophyll content in the composition is from 1 mg to 75 mg chlorophyll per g composition.

8. The use according to any one of the claims 1 to 7, wherein said use is for promoting lactic acid bacteria in the intestines of a mammal; and wherein the daily dose of thylakoids to be administered is 2.4 mg to 240 mg thylakoids per kg body mass of the mammal.

9. The use according to any of the preceding claims, wherein said composition is part of a foodstuff.

10. The use according to any of the preceding claims, wherein the composition is an oral nutraceutical composition comprising a physiologically tolerable oil-in-water emulsion comprising the thylakoids, or parts thereof.

11. The use according to claim 10, wherein the composition comprises 20 to 30 wt.% thylakoids and 20 to 30 wt.% oil.

12. A composition comprising isolated, enriched thylakoids, or parts thereof, and probiotic bacteria, wherein the chlorophyll content in the composition is from 1 mg to 75 mg chlorophyll per g composition.

13. The composition according to claim 12, wherein the probiotic bacteria are *Lactobacillus, e.g. Lactobacillus plantarum,* or *Bifidobacterium, e.g. Bifidobacterium infantis.*

14. The composition according to any one of the claims 12 or 13, wherein the thylakoids are enriched from green leaves or green algae; preferably from spinach.

15. The composition according to any one of the claims 12 to 14, wherein the composition is an oral pharmaceutical or nutraceutical composition comprising a physiologically tolerable oil-in-water emulsion comprising thylakoids; preferably the composition comprises 20 to 30 wt.% thylakoids and 20 to 30 wt.% oil.

**Patentansprüche**

1. Nicht-therapeutische Verwendung einer Zusammensetzung umfassend isolierte, angereicherte Thylakoide oder Teile derselben als präbiotisches Mittel.

2. Die Verwendung gemäß Anspruch 1, wobei diese Verwendung zur Förderung von Milchsäurebakterien im Darm, wie z. B. dem Ileum, eines Säugetiers ist.

3. Die Verwendung gemäß Anspruch 2, wobei die zu fördernden Bakterien *Lactobacilli* und/oder *Bifidobacterium* sind.

4. Die Verwendung gemäß einem der Ansprüche 1 bis 3, wobei die Zusammensetzung weiterhin probiotische Bakterien umfasst, wie z. B. *Bifidobacterium,* z. B. *Bifidobacterium infantis* oder *Lactobacillus,* z. B. *Lactobacillus plantarum.*

5. Die Verwendung gemäß einem der vorhergehenden Ansprüche, wobei die Thylakoide oder Teile derselben aus grünen Blättern oder Grünalgen angereichert sind.

6. Die Verwendung gemäß Anspruch 5, wobei die Thylakoide oder Teile derselben aus Spinat angereicherte Thylakoide oder Teile derselben sind.

7. Die Verwendung gemäß einem der vorhergehenden Ansprüche, wobei der Chlorophyllgehalt in der Zusammensetzung von 1 mg bis 75 mg Chlorophyll pro g der Zusammensetzung beträgt.

8. Die Verwendung gemäß einem der Ansprüche 1 bis 7, wobei diese Verwendung zur Förderung von Milchsäurebakterien im Darm eines Säugetiers ist und wobei die zu verabreichende tägliche Dosis an Thylakoiden 2,4 mg bis 240 mg Thylakoide pro kg Körpergewicht des Säugetiers beträgt.

9. Die Verwendung gemäß einem der vorhergehenden Ansprüche, wobei diese Zusammensetzung Teil eines Nahrungsmittels ist.

10. Die Verwendung gemäß einem der vorhergehenden Ansprüche, wobei die Zusammensetzung eine oral anwendbare, nutrazeutische Zusammensetzung ist, die eine physiologisch verträgliche Öl-in-Wasser-Emulsion umfassend die Thylakoide oder Teile derselben enthält.

11. Die Verwendung gemäß Anspruch 10, wobei die Zusammensetzung 20 bis 30 Gew.-% Thylakoide und 20 bis 30 Gew.-% Öl umfasst.

12. Eine Zusammensetzung umfassend isolierte, angereicherte Thylakoide oder Teile derselben und probiotische Bakterien, wobei der Chlorophyllgehalt in der Zusammensetzung von 1 mg bis 75 mg Chlorophyll pro g der Zusammensetzung beträgt.

13. Die Zusammensetzung gemäß Anspruch 12, wobei die probiotischen Bakterien *Lactobacillus,* z. B. *Lactobacillus plantarum* oder *Bifidobacterium,* z. B. *Bifidobacterium infantis* sind.

14. Die Zusammensetzung gemäß einem der Ansprüche 12 oder 13, wobei die Thylakoide aus grünen Blättern oder Grünalgen angereichert sind, vorzugsweise aus Spinat.

15. Die Zusammensetzung gemäß einem der Ansprüche 12 bis 14, wobei die Zusammensetzung eine oral anwendbare, pharmazeutische oder nutrazeutische Zusammensetzung ist, welche eine physiologisch verträgliche Öl-in-Wasser-Emulsion umfassend Thylakoide enthält, wobei die Zusammensetzung vorzugsweise 20 bis 30 Gew.-% Thylakoide und 20 bis 30 Gew.-% Öl umfasst.

**Revendications**

1. Utilisation non thérapeutique d'une composition comprenant des thylakoïdes enrichis isolés, ou des parties de ceux-ci, en tant qu'agent prébiotique.

2. Utilisation selon la revendication 1, dans laquelle ladite utilisation consiste à promouvoir des bactéries d'acide lactique dans les intestins, tels que l'iléon, d'un mammifère.

**3.** Utilisation selon la revendication 2, dans laquelle lesdites bactéries à promouvoir sont *Lactobacilli* et/ou *Bifidobacterium.*

**4.** Utilisation selon l'une quelconque des revendications 1 à 3, dans laquelle la composition comprend en outre des bactéries probiotiques telles que *Bifidobacterium,* par exemple *Bifidobacterium infantis,* ou *Lactobacillus,* par exemple *Lactobacillus plantarum.*

**5.** Utilisation selon l'une quelconque des revendications précédentes, dans laquelle les thylakoïdes, ou des parties de ceux-ci, sont enrichis en feuilles vertes ou en algues vertes.

**6.** Utilisation selon la revendication 5, dans laquelle lesdits thylakoïdes, ou des parties de ceux-ci, sont des thylakoïdes, ou des parties de ceux-ci, enrichis en épinard.

**7.** Utilisation selon l'une quelconque des revendications précédentes, dans laquelle la teneur en chlorophylle dans la composition est de 1 mg à 75 mg de chlorophylle par g de composition.

**8.** Utilisation selon l'une quelconque des revendications 1 à 7, dans laquelle ladite utilisation consiste à promouvoir des bactéries d'acide lactique dans les intestins d'un mammifère ; et dans laquelle la dose quotidienne de thylakoïdes à administrer est de 2,4 mg à 240 mg de thylakoïdes par kg de masse corporelle du mammifère.

**9.** Utilisation selon l'une quelconque des revendications précédentes, dans laquelle ladite composition fait partie d'un aliment.

**10.** Utilisation selon l'une quelconque des revendications précédentes, dans laquelle la composition est une composition nutraceutique orale comprenant une émulsion huile dans l'eau physiologiquement tolérable comprenant les thylakoïdes, ou des parties de ceux-ci.

**11.** Utilisation selon la revendication 10, dans laquelle la composition comprend 20 à 30 % en poids de thylakoïdes et 20 à 30 % en poids d'huile.

**12.** Composition comprenant des thylakoïdes enrichis isolés, ou des parties de ceux-ci, et des bactéries probiotiques, dans laquelle la teneur en chlorophylle dans la composition est de 1 mg à 75 mg de chlorophylle par g de composition.

**13.** Composition selon la revendication 12, dans laquelle les bactéries probiotiques sont *Lactobacillus,* par exemple *Lactobacillus plantarum,* ou *Bifidobacterium,* par exemple *Bifidobacterium infantis.*

**14.** Composition selon l'une quelconque des revendications 12 ou 13, dans laquelle les thylakoïdes sont enrichis en feuilles vertes ou en algues vertes ; de préférence, en épinard.

**15.** Composition selon l'une quelconque des revendications 12 à 14, dans laquelle la composition est une composition nutraceutique ou pharmaceutique orale comprenant une émulsion huile dans l'eau physiologiquement tolérable comprenant des thylakoïdes ; de préférence la composition comprend 20 à 30 % en poids de thylakoïdes et 20 à 30 % en poids d'huile.

Fig. 1

Fig. 2

Fig. 3

Fig. 4

Fig. 5

EP 2 895 012 B1

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1056358 B1
- US 2008175899 A1
- WO 2012113918 A1
- CN 101278752 A
- WO 2006132586 A
- WO 2010008333 A
- SE 2006000676 W
- WO SE2009000327 A
- SE 12510194

### Non-patent literature cited in the description

- **EMEK, SC ; SZILAGYI A ; AKERLUND H E et al.** A large scale method for preparation of plant thylakoids for use in body weight regulation. *Prepar biochem & biotech,* 2010, vol. 40 (1), 13-27
- **MONTELIUS C ; GUSTAFSSON K ; WESTROM B et al.** Thylakoids reduce glucose uptake and decrease intestinal macromolecular permeability. *Br J Nutr,* 2011, vol. 106 (6), 836-44